(19) Europäisches Patentamt
European Patent Office
Office européen des brevets

(11) EP 4 079 327 A1

(12) EUROPEAN PATENT APPLICATION

(43) Date of publication:
**26.10.2022 Bulletin 2022/43**

(21) Application number: **21305529.6**

(22) Date of filing: **22.04.2021**

(51) International Patent Classification (IPC):
***A61K 47/64*** *(2017.01)* ***A61K 47/65*** *(2017.01)*
***A61K 47/68*** *(2017.01)* ***A61P 35/00*** *(2006.01)*

(52) Cooperative Patent Classification (CPC):
**A61K 47/64; A61K 47/65; A61K 47/6803; A61K 47/6809; A61K 47/6889; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Centaurus Polytherapeutics**
**75005 Paris (FR)**

(72) Inventor: **Skerdi, HAVIARI**
**PARIS (FR)**

(74) Representative: **Cabinet Becker et Associés**
**25, rue Louis le Grand**
**75002 Paris (FR)**

# (54) PAYLOADS FOR DRUG-CONJUGATES AND THEIR USE FOR TREATING CANCER

(57) The present invention relates to a drug conjugate of the following formula (I):

$$Bd(L-D)n \quad (I)$$

wherein,
D is a drug moiety D is a drug moiety deriving from a cytotoxic drug selected from the group consisting of Ombrabulin (II), N-[[(2,2-Diacetoxyethyl)oxy]ethyl]doxorubicin (III), N-(2-chlorobenzyl)-10-methylaminocolchicine (IV), Mitomycin A (V), BMS-310705 (VI), and HTI-042 (VII), wherein the point of attachment with L is the nitrogen atom of the amino group or the oxygen atom of the hydroxyl group marked with **, and derivatives thereof;
L is a cleavable linker comprising a self-immolative moiety ($L_{SI}$) and an enzyme-cleavable unit ($L_C$);
Bd is a binder selected from the group consisting of the molecules that bind to a targeted antigen such as an antigen selected from the group consisting of proteins, glycoproteins, glycolipids, carbohydrates, or a combination thereof; and
n is an integer comprised from 1 to 4;
or a pharmaceutically acceptable salt thereof.

EP 4 079 327 A1

## Description

### FIELD OF THE INVENTION

The present invention relates to specific drug conjugates, and their use for treating cancer.

### TECHNICAL BACKGROUND

Cancers are among the most frequent illnesses nowadays. In particular, cancer is one of the most important causes of death in industrialized countries, and the number of people diagnosed with cancer continues to grow at the current time.

Several treatment modes have been developed so far, however chemotherapy has dominated the field for a long time. There are many chemotherapy drugs available. They directly destroy cancer cells or prevent them from multiplying.

In recent years, drug conjugates and more specifically antibody-drug conjugates (ADCs) have been developed as targeted therapy for treating cancer. In contrast to chemotherapy, drug conjugates are intended to target and kill tumor cells while sparing healthy cells. Drug conjugates are thus considered a more efficacious and safer cancer treatment and have gained increasing interest. Drug conjugates are generally composed of three parts: the binder part, the linker part and the payload part. The binder part specifically attaches to cancer cells by targeting tumor-specific antigens that are abundant in tumor tissue but minimally expressed in normal tissues. The binder is usually a protein, such as an antibody. The linker part holds the payload inactive until it reaches cancer cells, it thus ensures that less of the cytotoxic payload falls off before reaching a tumor cell, improving safety, and limiting dosages. The linker is thus stable in blood circulation and generally cleaved in tumor tissue by reduction, low pH, enzyme or antibody degradation. The payload part is intended to be released directly on cancer cells to kill them. The payload is usually a biological active cytotoxic anticancer drug that comprises appropriate conjugate site for linker attachment. One of the main prerequisites for the activity of such conjugates is the efficient release of the cytotoxic drug (payload) once the conjugate is bound to cancer cells, while the linker between the binder and payload should be stable in blood circulation before the conjugate reaches its biological target.

To date, a few ADCs have received market approval, all for treating cancer. However, when treating cancer, there are many criteria that must be considered in the choice of the drug (type of cancer, evolution stage, location of the tumor, conditions of the patient...). In addition, in the event of a recurrence or resistance to a particular drug, a different drug treatment should also be proposed as second line therapy. It is therefore important that a large number of possible treatments be proposed.

In recent years, clinical studies have been conducted for identifying new drug conjugates, in particular as second line therapy for recurrent or metastatic cancer, e.g. Zoptarelin conjugates, Milatuzumab-Doxorubicin conjugates or Vintafolide conjugates. However, these clinical studies have been stopped in absence of efficacy.

There is thus a need for developing drug conjugates with existing payloads that have shown great results for treating cancer standalone.

Ombrabulin (AVE8062) is a derivative of CA-4 phosphate, which is known to exhibit antivascular effects through selective disruption of the tubulin cytoskeleton of endothelial cells. In addition to antivascular effects on endothelial cells, it has been shown that AVE8062 have direct cytotoxic effects on tumor cells. In particular, Ombrabulin inhibits ovarian cancer growth through direct effects on endothelial cells causing substantive disruption in tumor blood flow leading to central necrosis in orthotopic murine model *(*Tae Jin Kim et al., Cancer Research 2007; 67: (19). October 1, 2007*)*.

N-[[(2,2-Diacetoxyethyl)oxy]ethyl]doxorubicin, abbreviated herein as DAEDOX, is an intensely cytotoxic doxorubicin analogue that retains full potency against tumor cells that express elevated levels of P-glycoprotein and are resistant to doxorubicin *(*David Farquhar et al., J. Med. Chem. 1998, 41, 965-972)*.*

Colchicine is a well-known anticancer compound showing antimitotic effect on cells. Its high cytotoxic activity against different cancer cell lines has been demonstrated many times. Colchicine analogs have been developed such as N-(2-chlorobenzyl)-10-methylaminocolchicine (abbreviated herein as CBACOL). CBACOL was shown to be active at nanomolar concentrations and exhibited better antiproliferative activity than the commonly used anticancer agents doxorubicin and cisplatin (Julia Krzywik and al., Molecules 2020, 25, 3540*)*.

Mitomycin A is a drug analog of the well-known antitumor agent Mitomycin C. Mitomycin A is an antitumor agents that is both more easily reduced and more toxic than mitomycin C *(*Kenneth R. Kunz et al., J. Med. Chem. 1991, 34, 2281-2286*;* and Robert T. Dorr, Cancer Chemother Pharmacol (1992) 31:1-5*)* .

BMS-310705 is an aqueous soluble semi-synthetic analogue of epothilone B that was selected for clinical development because of *in vivo* anti-tumour activity similar to that of optimally dosed ixabepilone in a series of human tumour xenografts resistant to paclitaxel through different mechanisms, such as the ovarian Pat-7, the pancreatic Pat-26 and the ovarian A2780 carcinoma. Importantly, the *in vivo* activity was reported at doses below the maximum tolerated

dose (MTD).The toxicity profile was comparable to that of ixabepilone, while the improved solubility allowed a cremophore-free formulation thus possibly avoiding pre-medication (C. Sessa and al., Annals of Oncology 18: 1548-1553, 2007*).*

HTI-042 has been developed as an inhibitor of tubulin polymerisation, for the treatment of advanced malignant solid tumors *(*Zask et al., J Med Chem 47, 4774-4786, 2004*).*

These six antitumor agents have never been developed as payload in drug-conjugates.

## SUMMARY OF THE INVENTION

The inventors have found six known antitumor drugs with high potency that can be used as payload in drug-conjugates. These drugs are in particular: Ombrabulin (AVE8062), N-[[(2,2-Diacetoxyethyl)oxy]ethyl]doxorubicin (also called DAEDOX), N-(2-chlorobenzyl)-10-methylaminocolchicine (also called CBACOL), Mitomycin A, BMS-310705, and HTI-042.

One aspect of the invention is thus a drug conjugate of the following formula (I):

$$Bd(L\text{-}D)_n \qquad (I)$$

wherein,

D is a drug moiety deriving from a cytotoxic drug selected from the group consisting of cytotoxic drugs of the following formula (II) to (VII):

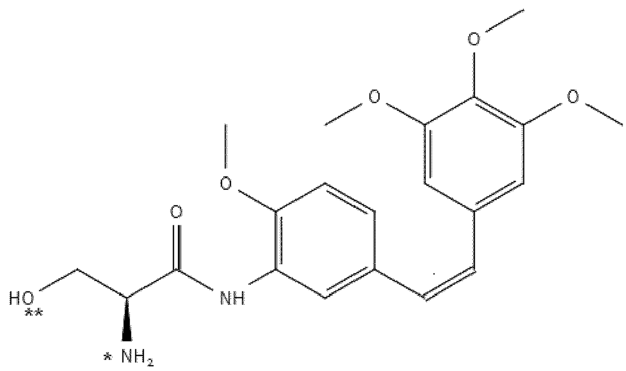

(II);

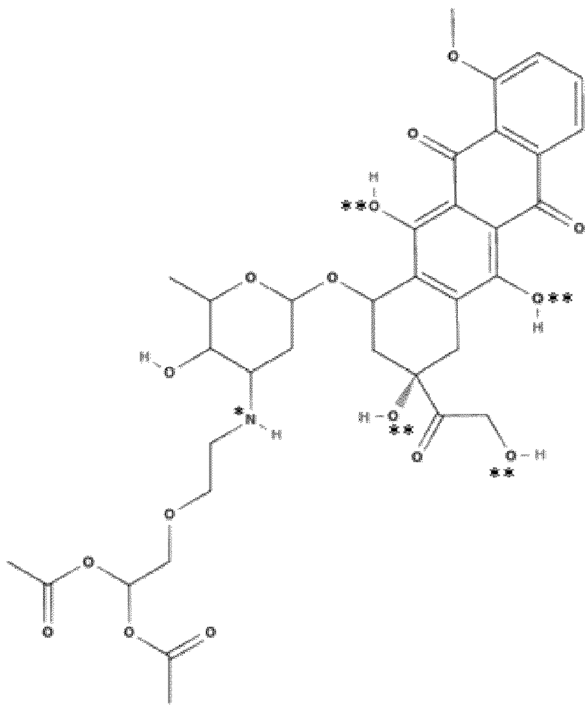

(III);

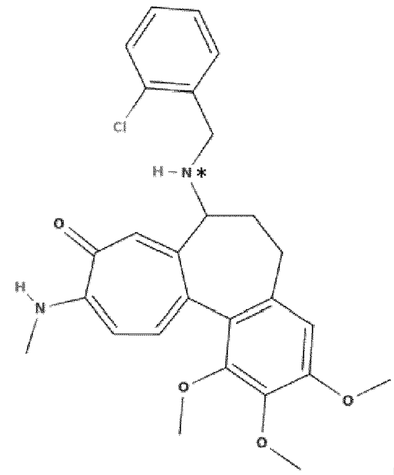

(IV);

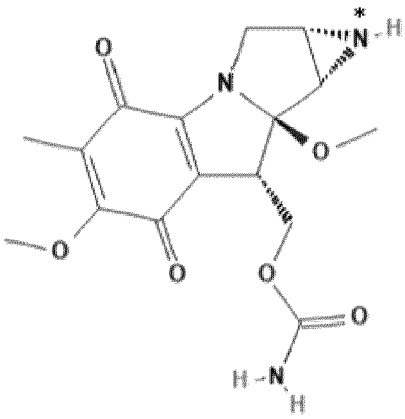

(V);

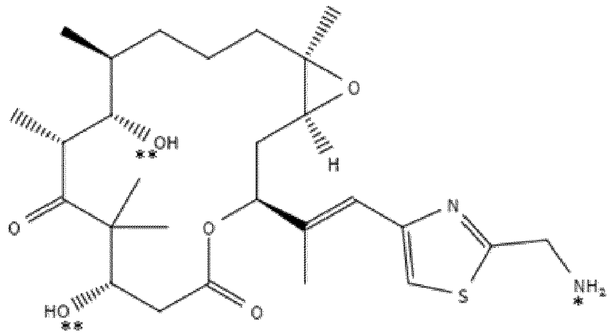

(VI);

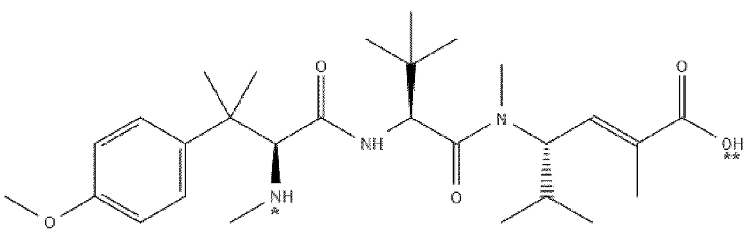

(VII),

wherein the point of attachment with L is the nitrogen atom of the amino group marked with * or the oxygen atom of the hydroxyl group marked with **, and derivatives thereof,

L is a cleavable linker comprising a self-immolative moiety ($L_{SI}$) and an enzyme-cleavable unit ($L_C$); Bd is a binder

selected from the group consisting of the molecules that bind to a targeted antigen such as an antigen selected from the group consisting of proteins, glycoproteins, glycolipids, carbohydrates, or a combination thereof; and
n is an integer comprised from 1 to 4;
or a pharmaceutically acceptable salt thereof.

Another aspect of the invention is the drug conjugate of the invention for use as a medicament, in particular for treating cancer.

Another aspect of the invention is a pharmaceutical composition comprising the drug conjugate of the invention, and a pharmaceutically acceptable excipient.

Another aspect of the invention is the pharmaceutical composition of the invention, for use for treating cancer.

## DETAILED DESCRIPTION OF THE INVENTION

### Definitions

In the present invention, by "pharmaceutically acceptable" is meant that which can be used in the preparation of a pharmaceutical composition which is generally safe, non-toxic and neither biologically nor otherwise undesirable, and which is acceptable for veterinary use as well as for human pharmaceutical use.

By "pharmaceutically acceptable salt" of a compound is meant a salt which is pharmaceutically acceptable as defined herein and which has the desired pharmacology activity of the parent compound. Pharmaceutically acceptable salts notably comprise:

(1) the addition salts of a pharmaceutically acceptable acid formed with pharmaceutically acceptable inorganic acids such as hydrochloric, hydrobromic, phosphoric, sulfuric and similar acids; or formed with pharmaceutically acceptable organic acids such as acetic, trifluoroacetic, propionic, succinic, fumaric, malic, tartaric, citric, ascorbic, maleic, glutamic, benzoic, salicylic, toluenesulfonic, methanesulfonic, stearic, lactic and similar acids; and
(2) the addition salts of a pharmaceutically acceptable base formed when an acid proton present in the parent compound is either replaced by a metallic ion e.g. an alkaline metal ion, an alkaline-earth metal ion or an aluminium ion; or coordinated with a pharmaceutically acceptable organic base such as lysine, arginine and similar; or with a pharmaceutically acceptable inorganic base such as sodium hydroxide, potash, calcium hydroxide and similar.

These salts can be prepared from the compounds of the invention containing a base or acid function, and the corresponding acids or bases using conventional chemical methods

In the context of the invention, a "peptide" refers to short chains of between 2 and 50 amino acids, linked by peptide bonds. Consequently, a "(X-Y)amino-acid peptide" refers to a peptide as defined above, comprising from X to Y amino-acid units, X and Y being integers. In addition, a "Cyclic (X-Y)amino-acid peptide" refers to a peptide comprising from X to Y amino-acid units, X and Y being integers, and comprising a circular sequence of bonds. The circular sequence of bonds can be through a connection between the amino and carboxyl ends of the peptide, a connection between the amino end and a side chain, a connection between the carboxyl end and a side chain, or a connection between two side chains.

In the context of the invention, a "protein" refers to one or more polypeptides arranged in a biologically functional way. Polypeptides are generally long chains of more than 50 amino acids, linked by peptide bonds.

In the context of the invention, an "alkyl group" refers to a straight or branched, saturated hydrocarbon chain. For example, mention can be made of methyl, ethyl, n-propyl, iso-propyl, n-butyl, iso-butyl, sec-butyl, t-butyl, n-pentyl, iso-pentyl, sec-pentyl, tert-pentyl, n-hexyl, iso-hexyl, sec-hexyl, tert-hexyl, and the like. By "(Cx-Cy)alkyl" in the present invention is meant an alkyl chain such as defined above comprising x to y carbon atoms. Therefore, a (C1-C6)alkyl group is an alkyl chain having 1 to 6 carbon atoms.

The term "aryl", as used in the present invention, refers to an aromatic hydrocarbon group comprising preferably 6 to 10 carbon atoms and comprising one or more, notably 1 or 2, fused rings, such as, for example, a phenyl or naphtyl group.

The term "heteroaryl" as used in the present invention refers to an aromatic hydrocarbon monocycle or polycycle (comprising fused, bridged or spiro rings), in which one or more, advantageously 1 to 4, and more advantageously 1 or 2, carbon atoms have each been replaced with a heteroatom selected from nitrogen, oxygen and sulphur atoms. Advantageously, the heterocycle comprises 5 to 15, notably 5 to 10 atoms in the ring.

The term "halogen" or "halo", as used in the present invention, refers to a fluorine, bromine, chlorine or iodine atom.

The term "hydroxyl group", as used in the present invention, refers to a group -OH.

## Payloads

In general formula (I) of the invention, D is a drug moiety deriving from a cytotoxic drug selected from the group consisting of the cytotoxic drugs of the formula (II) to (VII) above identified, wherein the point of attachment with the cleavable linker (L) is the nitrogen atom of the amino group marked with * or the oxygen atom of the hydroxyl group marked with **, and derivatives thereof, or a pharmaceutically acceptable salt thereof. Advantageously, the point of attachment with the cleavable linker (L) is the nitrogen atom of the amino group marked with *.

The compounds of general formula (II) to (VII) may be in the form of a derivative thereof. In the context of the invention, a derivative of a compound has a chemical structure very related to the compound and displays similar pharmacokinetic and pharmacodynamic properties. Structural differences between said derivative and the compound may lie in the addition of any suitable substituent commonly found in the art. For example, the substituent may be an alkyl group, aryl group, heteroaryl group or an halogen atom, preferably a (C1-C6)alkyl group or an halogen atom. The structural difference may also consist of modification the N-terminal position so as to render this function compatible with the establishment of a covalent bond with the linker.

According to the invention, a "drug moiety", also called payload, is a radical deriving from a cytotoxic drug, i.e. a moiety consisting of a cytotoxic drug deprived of one or more atoms of one of its functional groups (for instance, a H atom) which has reacted with the remainder of the conjugate.

The drug moiety according to the invention is deriving from a cytotoxic drug. As used herein, the term "cytotoxic drug" refers to a molecule that when entering in contact with a cell, optionally upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins. In principle, a cytotoxic drug is defined as a LO1 ATC molecule ("Anatomical Therapeutic Chemical Classification System" where LO1 is a subgroup defining antineoplastic and immunomodulating agents defined by WHO Collaborating Centre for Drug Statistics Methodology).

Advantageously, the cytotoxic drug is selected from the group consisting of Ombrabulin (AVE8062) of formula (II), N-[[(2,2-Diacetoxyethyl)oxy]ethyl]doxorubicin (also called DAEDOX) of formula (III), N-(2-chlorobenzyl)-10-methylaminocolchicine (also called CBACOL) of formula (IV), Mitomycin A of formula (V), BMS-310705 of formula (VI), and HTI-042 of formula (VII).

In one aspect, the cytotoxic drug of formula (II) is Ombrabulin (AVE8062) or a pharmaceutically acceptable salt thereof, and is in particular in the form of a hydrochloride salt. In particular, Ombrabulin is named as (2S)-2-Amino-3-hydroxy-N-[2-methoxy-5-[(1Z)-2-(3,4,5-trimethoxyphenyl)ethenyl]phenyl]-propanamide hydrochloride (CAS number 253426-24-3).

In one aspect, the cytotoxic drug of formula (III) is N-[[(2,2-Diacetoxyethyl)oxy]ethyl]doxorubicin (also called DAEDOX), or a pharmaceutically acceptable salt thereof.

In one aspect, the cytotoxic drug of formula (IV) is N-(2-chlorobenzyl)-10-methylaminocolchicine (also called CBACOL), or a pharmaceutically acceptable salt thereof.

In one aspect, the cytotoxic drug of formula (V) is Mitomycin A (CAS Number 4055-39-4), or a pharmaceutically acceptable salt thereof.

In one aspect, the cytotoxic drug of formula (VI) is BMS-310705, also called 21-Aminoepothilone B (CAS Number 280578-49-6), or a pharmaceutically acceptable salt thereof.

In one aspect, the cytotoxic drug of formula (VII) is HTI-042, or a pharmaceutically acceptable salt thereof.

In a particular embodiment, D is selected from the group consisting of drug moieties of the formula (II), and (IV) to (VII) above identified and derivatives thereof, and the drug moiety of the formula (III), or a pharmaceutically acceptable salt thereof. In particular, D is selected from the group consisting of drug moieties of the formula (IV) to (VI) above identified and derivatives thereof, and the drug moieties of the formula (II) and (III), or a pharmaceutically acceptable salt thereof. More particularly, D is selected from the group consisting of drug moieties of the formula (IV) to (VI) above identified and derivatives thereof, or a pharmaceutically acceptable salt thereof, preferably of the formula (V) or (VI) and derivatives thereof, or a pharmaceutically acceptable salt thereof.

Advantageously, D is selected from the group consisting of drug moieties of the formula (II) to (VII), more advantageously (II), and (IV) to (VI) above identified, or a pharmaceutically acceptable salt thereof. More advantageously, D is selected from the group consisting of drug moieties of the formula (V) or (VI) above identified or a pharmaceutically acceptable salt thereof, preferably of the formula (VI), or a pharmaceutically acceptable salt thereof.

## Linker

In general formula (I) of the invention, L is a cleavable linker comprising a self-immolative moiety ($L_{SI}$) and an enzyme-cleavable unit ($L_C$);
In the context of the invention, a "linker" is a bifunctional moiety that can be used to link one drug moiety (payload) to a

binder Bd, to form a drug-conjugate of general formula (I).

In the context of the invention "cleavable linker (L)" is a linker as defined above that cleaves for selectively releasing the payloads in or near the targeted cells. Cleavable linker uses the inherent properties of targeted cells (e.g. tumor cells) to selectively release the payloads. Cleavable linkers are stable in the blood circulation and generally release the payload using one of the following mechanisms: 1) enzyme sensitivity, 2) pH sensitivity, and 3) glutathione sensitivity. For example, the cleavable linker may be an acid-labile linker, peptidase-sensitive linker, photolabile linker, dimethyl linker or disulfide-containing linker. The linker is, in a particular embodiment, cleavable under intracellular conditions, such that cleavage of the linker releases the drug from the binder in the intracellular environment.

According to the invention, the cleavable linker (L) is cleavable via enzyme degradation and may comprise or may consist of, for example, a peptidyl linker that is cleaved by an enzyme such as a protease (also called peptidase), including, but not limited to, a lysosomal or endosomal protease. In particular, cleaving agents can include cathepsin, in particular cathepsins B and D, or plasmin, all of which are known to hydrolyze dipeptide drug derivatives resulting in the release of active drug inside or near target cells.

In an advantageous embodiment of the invention, the linker L is of the following formula (VIII):

$$*(-L_C-L_{SI}-** \qquad \text{(VIII)},$$

wherein the binder Bd is linked to $-L_A-$ via the bond identified with *, and D is linked to $-L_{SI}-$via the bond identified with **; and wherein

$-L_A-$ represents an anchor unit, a being 0 or 1;

$-L_S-$ represents a spacer unit, s being an integer from 0 to 15;

- r is an integer from 0 to 10;

$-L_C-$ represents an enzyme-cleavable unit; and

$-L_{SI}-$ represents a self-immolative moiety.

In the context of the invention, a "self-immolative moiety ($L_{SI}$)" refers to a divalent chemical group which connects a drug moiety (payload) with the remainder of the conjugate, and which becomes labile upon activation (e.g. enzymatic cleavage), leading to a release of the free moieties, in particular the payload. Self-immolative moieties can thus release the drug without the need for a separate hydrolysis step. Self-immolative moieties are well-known to the skilled artisan (Polym. Chem. 2011, 2, 773-790).

In particular, the self-immolative moiety ($L_{SI}$) is selected form the group consisting of residue of *p*-aminobenzyl alcohol (PAB) unit, residue of 2-aminoimidazol-5-methanol derivatives, hemithioaminal derivatives of para-carboxybenzaldehyde or glyoxilic acid, bis-amine cyclizers, lactam cyclizers (*as described in* Yves Meyer et al. Org. Biomol. Chem., 2010, 8, 1777-1780*)*, and *ortho*- or *para*-aminobenzylacetals.

In an advantageous embodiment of the invention, $L_{SI}$ is a p-aminobenzylcarbonyl (PAB-carbonyl) unit, in particular $-L_{SI}-$ is represented by the following formula (IX):

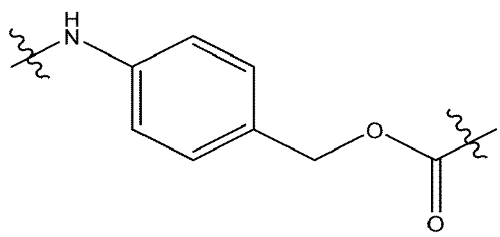
(IX),

wherein $-L_{SI}-$ is linked to $-L_C-$via the aniline group and $-L_{SI}-$ is linked to D via the carbonyl group.

In the context of the invention, a "enzyme-cleavable unit ($L_C$)" refers to a group which is cleavable by an enzyme, in particular by a protease (also called peptidase), including, but not limited to, a lysosomal or endosomal protease. In particular, $-L_C-$ is an enzyme cleavable unit that is cleavable by a cathepsin enzyme or plasmin, in particular cathepsins A, B, C D, F, H, K or L, preferably cathepsin B.

Advantageously, $-L_C-$ comprises at least one amino acid unit, preferably at least two amino acid units, more preferably from 2 to 4 amino acid units, preferably selected from the group consisting of glycine (Gly), valine (Val), citrulline (Cit), phenylalanine (Phe), alanine (Ala) and arginine (Arg). In particular, $-L_C-$ comprises two, three or four amino acid units selected from the group consisting of glycine (Gly), valine (Val), citrulline (Cit), phenylalanine (Phe), alanine (Ala), lysine (Lys), and arginine (Arg), advantageously $-L_C-$ is -Ala-Ala-, -Val-Ala-, -Val-Cit-, -Val-Arg-, -Phe-Cit-, -Phe-Arg-; -Ala-Cit, -Ala-Arg-, -Phe-Leu-, -Phe-Lys-, -Gly-Phe-Leu-Gly-, or -Gly-Gly-Phe-Gly-, more advantageously $-L_C-$ is -Val-Cit-:

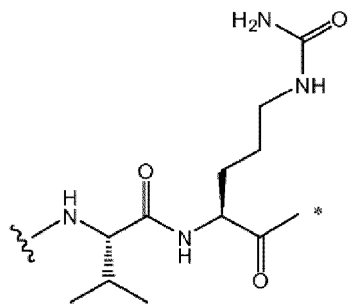

(Val-Cit),

wherein $-L_C-$ is linked to $-L_S-$ via the bond marked with a wavy line and $-L_C-$ is linked to $-L_{SI}-$ via the bond marked with an asterisk.

In another aspect,, the $-L_C-L_{SI}-$ unit may be selected from the group consisting of units of the following formula (X) to (XVII):

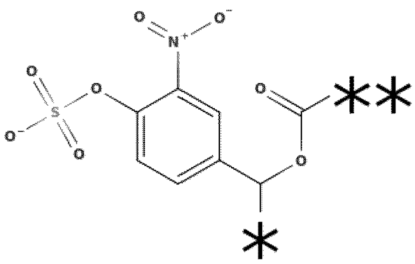

(X)

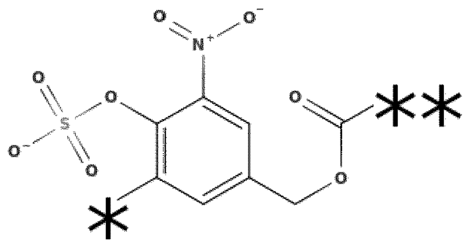

(XI)

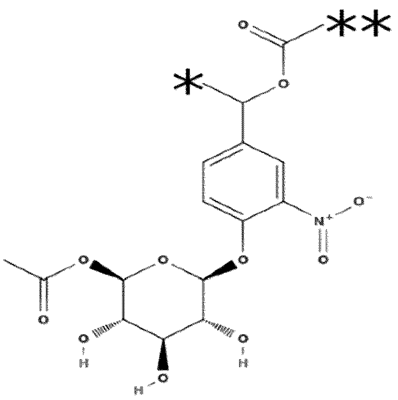

(XII)

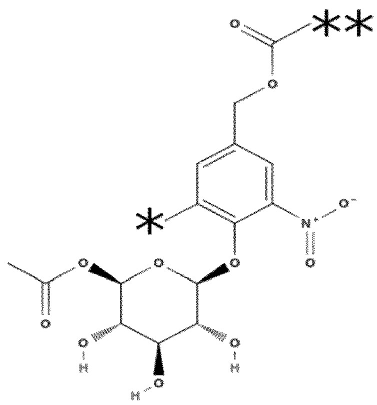

(XIII)

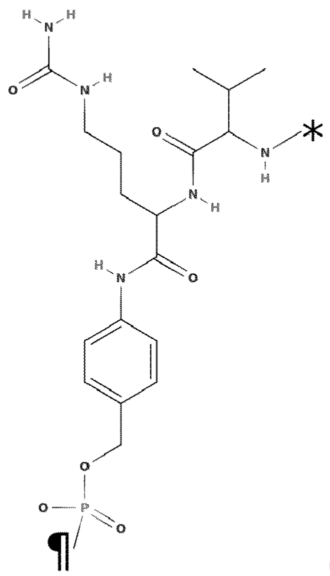

(XIV)

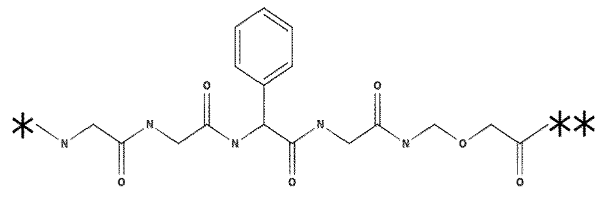

(XV)

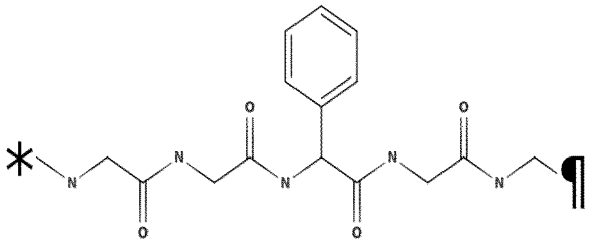

(XVI)

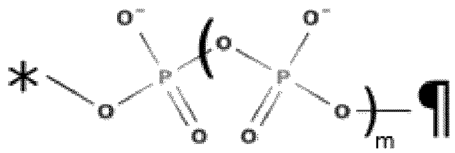

(XVII)

wherein, the nitro ($-NO_2$) group, when present, may be replaced with a hydrogen atom (H), and wherein * refers to the unit Bd-$((L_A)_a$-$(L_S)_s$-$)_r$, ** refers to either the payload (-D) or the payload with an additional self-immolative unit (-$L_{SI}$'-D) bound through an amine group, refers to either the payload (-D) or the payload with an additional self-immolative unit (-$L_{SI}$'-D) bound through a hydroxy group, when one is present, and wherein m is 0 to 3. Advantageously, ** and refer to the payload (-D).

In the context of the invention, a "spacer unit ($L_S$)" refers to a linear or branched chain of atoms whose purpose is to provide flexibility and distance between the chemical groups at its ends and/or to improve solubility in water and/or to facilitate chemical accessibility of reactive groups at its ends, and that is stable in bodily fluids.

Advantageously, -$L_S$- represents a unit selected in the group consisting of sugar moieties, ethylene glycol, aliphatic chain comprising from 0 to 30 carbon atoms, 1-amino-1-glucitolyl-y-glutamate, amino-acids, and homo- and hetero-polymers thereof, either linear or branched. More advantageously, -$L_S$- represents a unit selected in the group consisting of 0 to 15 ethylene glycol units and/or 0 to 15 sarcosine units.

In the context of the invention, an "anchor unit ($L_A$)" refers to the result of a reaction between a chemical group that is capable of reacting and forming a highly stable chemical bond with a compatible free chemical group present on the standalone binder, either naturally or added through engineering, such reaction resulting in the $L_A$ moiety and the Bd-$L_A$ bond.

In one aspect, the anchor unit $L_A$ is the product of a reaction between an electrophile group and a nucleophile group (either one on the standalone binder Bd, and the other on the standalone linker L). Exemplary such electrophile groups include, but are not limited to, aldehyde and ketone carbonyl groups, maleimides, halomaleimides, alkynes, haloacetamides, a-haloacetyl, pyridyl disulfide, activated esters such as succinimide esters, N-hydroxysuccinimide, 4-nitrophenyl esters, pentafluorophenyl esters, tetrafluorophenyl esters, anhydrides, acid chlorides, sulfonyl chlorides, isocyanates, and isothiocyanates. Exemplary such nucleophile groups include amine, thiol, azide, hydroxyl, aminooxy, hydrazine, thiosemicarbazide (Stephanopoulos and Francis, Nat. Chem. Biol. 2011 Nov 15;7(12):876-84; Kalia and Raines Curr Org Chem. 2010 Jan; 14(2): 138-147).

Advantageously, -$L_A$- is or comprises a unit selected in the group consisting of maleimidocaproyl, amide, alkane diamides, alkanedioic acid esters, triazole, disulfide, oxime, hydrazone, disulfide.

Advantageously, in the formula (VIII) as defined below, r is an integer from 1 to 5, in particular r is 1 or 2.
Advantageously, in the formula (VIII) as defined below, s is an integer from 1 to 8, in particular from 1 to 4.
Advantageously, in the formula (VIII) as defined below, a is 1.
Advantageously, in the formula (I) as defined below, n is an integer from 1 to 3, in particular 1 or 2.

Binder

In the general formula (I), Bd represents the binder part of the drug conjugate. The binder part specifically attaches to a targeted antigen such as tumor-specific antigens. According to the invention, the binder is a molecule that binds to a targeted antigen such as an antigen selected from the group consisting of proteins, glycoproteins, glycolipids, carbohydrates, or a combination thereof.
In the context of the invention, "antigen" refers to molecule that are present on the outside of a cell. An antigen generally has numerous binding sites, also called epitopes, recognized by ceomplementarity-determining regions on multiple antibodies. Each antibody that specifically binds to a different epitope has a different structure. Thus, one antigen may have more than one corresponding antibody. An antibody includes a full-length immunoglobulin molecule or an immunologically active portion of a full-length.
In particular, the binder is selected from those which bind specifically a molecule present at the membrane of a cancer cell, i.e. a cancer cell antigen, preferably a molecule (antigen) selected from the group consisting of proteins, glycoproteins, glycolipids, carbohydrates, or a combination thereof, even more preferably the binder binds specifically an antigen present at the membrane of a cancer cell. The membrane molecule to which the binder is capable to bind to is a molecule (antigen) abundant at the membrane of a cancer cell. In a particular embodiment, the membrane molecule recognized by the binder is an antigen overexpressed at the membrane of a cancer cell. In addition, the binder can be bound to one or more linker moieties, to form a drug conjugate of general formula (I).
According to the invention, the binder is advantageously selected from the group consisting of antibodies, antibody fragments, peptides, and affibodies.
In the context of the invention, "antibody" refers to immunoglobulin molecules and immunologically active portions of immunoglobulin molecules, i.e., molecules that contain an antigen-binding site that immunospecifically binds an antigen. As such, the term antibody encompasses not only whole antibody molecules, but also antigen-binding antibody fragments as well as variants (including derivatives) of antibodies and antibody fragments. In particular, the antibody according to the invention may correspond to a monoclonal antibody (e.g. a chimeric, humanized or human antibody), or a fragment of monoclonal antibody. The term antibody refers to classical antibodies as well as to heavychain antibodies and fragments and derivatives thereof such as (VHH)2 fragments and single domain antibodies.
Antibodies according to the invention may be produced by any technique known in the art, such as, without limitation, any chemical, biological, genetic or enzymatic technique, either alone or in combination. The antibodies of the invention can be obtained by producing and culturing hybridomas. See also WO 2015/063331 for the production of synthetic single domain antibodies. The antibody according to the invention may be a monomeric antibody or a multimeric antibody and it may comprise at least a variable domain, in particular when the antibody is multimeric. Preferably, the antibody according to the invention is IgG, preferably subtypes IgG1 or IgG4, for example trastuzumab or rituximab.
In the context of the invention, a "antibody fragment", also called antigen-binding antibody fragments refers to a portion of a full length antibody, i.e. a peptide, polypeptide, or protein, retaining the ability to bind to the target (also generally referred as antigen) of the antibody, and comprising an amino acid sequence of at least 10 contiguous amino acid residues of the amino acid sequence of the antibody. Examples of antibody fragments include Fab, Fab', F(ab')2, and Fv fragments; diabodies; linear antibodies; minibodies (Olafsen et al.(2004) Protein Eng. Design & Sel. 17(4):315-323*),* fragments produced by a Fab expression library, anti-idiotypic (anti-Id) antibodies, CDR (complementary determining region), and epitope-binding fragments of any described herein which immunospecifically bind to cancer cell antigens, viral antigens or microbial antigens, single-chain antibody molecules; and multispecific antibodies formed from antibody fragments.
Antibody fragments according to the invention can be generated by known techniques. The antibody fragments are antigen binding portions of an antibody, such as F(ab')2, Fab, Fv, scFv and the like. Other antibody fragments include, but are not limited to: the F(ab')2 fragments which can be produced by pepsin digestion of the antibody molecule and the Fab' fragments, which can be generated by reducing disulfide bridges of the F(ab')2 fragments. Alternatively, Fab' expression libraries can be constructed (Huse et al., 1989, Science, 246:1274-1281) to allow rapid and easy identification of monoclonal Fab' fragments with the desired specificity.
In the context of the invention, "affibody" refers to robust proteins engineered to bind to target proteins or peptides with high affinity, imitating monoclonal antibodies. Typically, affibodies are 50-65 residue polypeptides that take a rigid three-helix conformation and bind their target thanks to their outward-facing residues.
In a most preferred embodiment of this invention, the binder Bd is a peptide, i.e. a short chain of 2 to 50 amino acids, linked by peptide bonds, that can be bound to a cancer cell antigen. Advantageously, the binder is selected from

the group consisting of a (7-25)amino-acid peptide, preferably a (10-20)amino-acid peptide, preferably a cyclic (10-20)amino-acid peptide. Advantageously, the binder is a cyclic (10-20)amino-acid peptide, preferably with a non-proteinogenic amino acid in C or N-terminal, in particular in C-terminal. Non-proteinogenic amino-acids are amino-acids which are not encoded in the canonical genetic code of RNA translation used by most living organisms, and thus have different biological properties than the 22 proteinogenic amino-acids, in particular bioorthogonal reactivities and/or resistance to enzymes. Preferred non-proteinogenic amino-acids for embodiments of this invention include beta amino-acids, D-amino-acids, sarcosine, azidolysine, and propargylglycine.

Process

The conjugate according to the invention may be prepared by any method known by the person skilled in the art such as, without limitation, i) reaction of a nucleophilic group of the binder with a bivalent linker reagent followed by reaction with a nucleophilic group of the drug, or ii) reaction of a nucleophilic group of the drug with a bivalent linker reagent followed by reaction with a nucleophilic group of the binder.

Nucleophilic groups on antibody include, without limitation, N-terminal amine groups, side chain amine groups (e.g. lysine), side chain thiol groups, and sugar hydroxyl or amino groups when the binder is glycosylated.

Nucleophilic groups on the drug include, without limitation, amine, thiol, and hydroxyl groups, and preferably amine groups.

Amine, thiol, and hydroxyl groups are nucleophilic and capable of reacting to form covalent bonds with electrophilic groups on linker moieties and linker reagents including, without limitation, active esters such as NHS esters, HOBt esters, halo formates, and acid halides; alkyl and benzyl halides such as haloacetamides; aldehydes; ketones; carboxyl; and maleimide groups. The binder may have reducible interchain disulfides, i.e. cysteine bridges.

In a preferred embodiment, the antibody-drug conjugate of the invention is prepared by preparation of the drug-linker moiety followed by coupling between a nucleophilic group of the binder (for ex. the SH group of a cysteine moiety) and an electrophilic group of the drug-linker moiety (for ex. a maleimide).

For example, the conjugate according to the invention may be prepared by carrying out the following steps:

*Step (a) Preparation of L-D*

All 6 drug moieties can be commercially found or can be obtained by applying methods known by the one skilled in the art and described in the literature. In particular, a method for preparing 21-aminoepothilone B is disclosed in US20030187039A1, a method for preparing HTI-042 is disclosed in US8129407B2 (similar method than the one disclosed in US8129407B2 for compounds ER-807842, ER-806005 or ER-806073) as well as in Niu et al. Bioorg Med Chem Lett 2010, 20, 1535-1538, a method for preparing Mitomycin A is disclosed in Vyas et al. J. Org. Chem. 1986, 51, 22, 4307-4309, a method for preparing Ombrabulin is disclosed in WO2003084919A2, a method for preparing DAEDOX is disclosed in Farquhar et al. J. Med. Chem. 1998, 41, 965-972, and a method for preparing CBACOL is disclosed in Krzywik et al. Molecules 2020, 25, 3540.

The Linker-Drug (L-D) moiety can be prepared by coupling:

- The linker L with the corresponding cytotoxic drug;
- A part of the linker L with the corresponding cytotoxic drug before completing the synthesis of the linker;
- The linker with a part or a precursor of the cytotoxic drug before completing the synthesis of the cytotoxic drug; or
- A part of the linker with a part or a precursor of the cytotoxic drug before completing the synthesis of the linker and the drug.

The coupling reactions are well known from the one skilled in the art between a nucleophilic group and an electrophilic group.

The nucleophilic group can be in particular an amine, thiol or hydroxyl group, and notably an amine or hydroxyl group. In a preferred embodiment it is a primary or secondary amine group.

The electrophilic group can be a carboxylic acid group (COOH) optionally in an activated form or an activated carbonate ester moiety, or an activated oxidized phosphorus.

By "activated form" of a carboxylic acid is meant a carboxylic acid in which the OH moiety of the COOH function has been replaced with an activated leaving group (LG) enabling coupling of the activated carboxylic acid group with an amino group in order to form an amide bond and release the compound LG-H. Activated forms may be activated esters, activated amides, anhydrides or acyl halides such as acyl chlorides. Activated esters include derivatives formed by reaction of the carboxylic acid group with N-hydroxybenzotriazole or N-hydroxysuccinimide.

By "activated carbonate ester" is meant a carbonate ester comprising a -OC(O)OR moiety in which OR represents a good leaving group enabling coupling of the activated carbonate ester with an amino group in order to form a

carbamate moiety and release the compound ROH. The R group of the activated carbonate ester includes, without limitation, the p-nitro-phenyl, pentafluorophenyl, 2,5-dioxo-2,5-dihydro-1H- pyrrol-1-yl and benzyl groups, preferably the p-nitro-phenyl and pentafluorophenyl groups.

By "activated form" of an oxidized phosphorus meant an oxidized phosphorus in which one or more of the oxygen atoms have been replaced with an activated leaving group (LG) enabling coupling of the activated phosphate group with an amino or hydroxy group in order to form a phosphoroamidite or phosphodiester bond and release the compound LG-H. Such leaving groups include diisopropylamine, 2-cyanoethanol, and trifluoromethanesulfonanilide.

*Step (c) Preparation of Bd-(L-D)n*

The complete Bd(-L-D)n conjugate can be prepared by coupling:

- The binder Bd with the Linker-Drug moiety or moeities;
- A part or precursor of the binder Bd with the Linker-Drug moiety or moieties before finishing the synthesis of the binder;
- The binder Bd with a part or a precursor of the Linker-Drug moiety or moieties before finishing synthesis of the Linker-Drug moiety or moieties;

The coupling reactions are well known from the one skilled in the art between a nucleophilic group and an electrophilic group.

Exemplary such electrophile groups include, but are not limited to, aldehyde and ketone carbonyl groups, maleimides, halomaleimides, alkynes, haloacetamides, a-haloacetyl, pyridyl disulfide, activated esters such as succinimide esters, N-hydroxysuccinimide, 4-nitrophenyl esters, pentafluorophenyl esters, tetrafluorophenyl esters, anhydrides, acid chlorides, sulfonyl chlorides, isocyanates, and isothiocyanates, preferrably maleimides, halomaleimides, carbonyls or alkynes. Exemplary such nucleophile groups include amine, thiol, azide, hydroxyl, aminooxy, hydrazine, thiosemicarbazide, preferrably thiol, amine and azide.

Each intermediate or product obtained at the end of a step may be isolated and optionally purified, or alternatively, several steps may be carried out one-pot without isolating said intermediate or product. The order of the process steps described below may be modified.

## Pharmaceutical compositions

Another aspect of the invention is a pharmaceutical composition comprising the drug-conjugate of the invention, and a pharmaceutically acceptable excipient.

The drug-conjugates of the invention may be delivered in a pharmaceutical composition comprising at least an effective amount of at least one drug-conjugate of the invention, and a pharmaceutically acceptable carrier.

As used herein, the term "pharmaceutically acceptable carrier" refers to any ingredient except active ingredients (namely excipients) that is present in a pharmaceutical composition. Its addition may be aimed at conferring a particular consistency or other physical or gustative properties to the final product. An excipient or pharmaceutically acceptable carrier must be devoid of any interaction, in particular chemical, with the actives ingredients. These pharmaceutically acceptable carrier and excipients are well known and will be adapted by the person skilled in the art as a function of the nature and of the mode of administration of the active compound(s) chosen.

According to the invention, the composition may be administered via oral route or via systemic route (subcutaneous, intradermal, intramuscular or intravenous). The mode of administration, dosage and optimum pharmaceutical forms can be determined according to the criteria generally taken into account in the establishment of a treatment adapted to a patient such as, for example, the age or the body weight of the patient, the seriousness of his/her general condition, the tolerance to the treatment and the secondary effects noted.

The pharmaceutical composition may be in a solid or liquid state and may be in any of the pharmaceutical forms commonly used in human and/or veterinary medicine, for example in the form of simple or coated tablets, of pills, of lozenges, of gel capsules, of drops, of powders (hard or soft gelatin capsule), of granules, of injectable preparations, of aqueous or non-aqueous solution, of ointments, of emulsion, of suspension, of creams or of gels. The pharmaceutical composition may be prepared according to the usual methods.

In the solid compositions, the active ingredient of the invention may be mixed with one or more inert diluents such as starch, cellulose, sucrose, lactose or silica, in a stream of argon. These compositions may also comprise substances other than diluents, for example one or more lubricants such as magnesium stearate or talc, a colouring agent, a coating (coated tablets) or a varnish.

The composition for systemic administration is sterile and may preferably be aqueous or non-aqueous solutions, suspensions or emulsions. As solvent or vehicle, use can be made of water, propylene glycol, a polyethylene glycol, vegetable oils, in particular olive oil, injectable organic esters e.g. ethyl oleate or other suitable organic solvents. These

compositions may also contain adjuvants, in particular wetting, isotonic, emulsifying, dispersing and stabilising agents. Sterilisation can be performed in several manners, for example by sanitising filtration, by incorporating sterilising agents into the composition, by radiation or by heating. They can also be prepared in the form of solid sterile compositions which can be dissolved at the time of use in sterile water or any other injectable sterile medium.

As used herein, the term "effective amount" refers to a quantity of an active ingredient effective, at dosages and for periods of time necessary, to achieve the desired therapeutic or prophylactic result, i.e. an amount effective for preventing, removing or reducing the deleterious effects of the disease.

Uses

Another aspect of the invention is the drug-conjugate of the invention or the pharmaceutical composition of the invention for use as a medicament, in particular for use for treating cancer.

In particular, the present invention is directed to a drug-conjugate of the invention or a pharmaceutical composition of the present invention for use for treating an antigen expressing cancer, said antigen being preferably selected from CD 19, CD20, CD22, CD25, CD30, CD33, CD40, CD56, CD64, CD70, CD74, CD79, CD105, CD138, CD174, CD205, CD227, CD326, CD340, MUC16, GPNMB, PSMA, Cripto, ED-B, TMEFF2, EphB2, EphA2, FAP, $\alpha$v integrin, Mesothelin, EGFR, TAG-72, GD2, CAIX, 5T4, HER1, HER3, HER2, IGF- 1R, Axl, Integrin a3, integrin b1, cadherin 6, claudin 16, claudin 1, SPINT2, BST, L1CAM, DCBLD2, BCAM, ANXA2, EPCAM, UPK3B, UPK1B, HLA-B, HLA-C, Trop-2 (also called TACSTD2), NaPi2b (also called SLC34A2), MET (also called HGFR), MSLN (also called mesothelin), MUC16 (also called CA125), and their extra cellular membrane (ECD) fragment, more preferably from the integrin a3, integrin b1, cadherin 6, claudin 16, claudin 1, DCBLD2, BCAM, and their extra cellular membrane (ECD) fragment, also more preferablyintegrin a3, integrin b1, BCAM, and their extra cellular membrane (ECD) fragment.

In an embodiment, said antigen-expressing cancer is a cancer chosen from breast, colon, esophageal carcinoma, hepatocellular, gastric, glioma, lung, melanoma, osteosarcoma, ovarian, prostate, rhabdomyosarcoma, renal, thyroid, uterine endometrial cancer, mesothelioma, oral squamous carcinoma, Kaposi sarcoma, acute leukemia, colorectal carcinoma, melanoma, pancreatic ductal adenocarcinoma and any drug resistant cancer.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

As used herein, the terms "subject", "individual" or "patient" are interchangeable and refer to an animal, preferably to a mammal, even more preferably to a human. However, the term "subject" can also refer to non-human animals, in particular mammals. The subject according to the invention is an animal, preferably a mammal, even more preferably a human. The subject may be a non-human animal, in particular selected from mammals such as dogs, cats, horses, cows, pigs, sheep and non-human primates, among others. Preferably, the subject is human.

The invention also relates to the use of a drug-conjugate according to the invention or a pharmaceutical composition according to the invention, for the preparation of a medicament, in particular for treating cancer, preferably an antigen-expressing cancer, in a subject in need thereof.

The invention further relates to a method for treating a cancer, in particular an antigen-expressing cancer, in a subject in need thereof, comprising administering to the subject, an effective amount of at least one drug-conjugate according to the present invention or of a composition according to the present invention.

The invention further relates to a method for treating a cancer, in particular an antigen-expressing cancer, in a subject in need thereof, comprising administering to the subject, an effective amount of at least one drug-conjugate according to the present invention or of a composition according to the present invention, in combination with another treatment chosen from a group consisting of chemotherapy, radiotherapy, treatment with at least one anti-inflammatory agent, and combinations thereof.

The drug-conjugate according to the invention or the pharmaceutical composition according to the invention may be administered by any convenient route to a subject in need thereof. For instance, it can be administered by a systemic route, in particular by subcutaneous, intramuscular, intravenous or intradermal, preferably by intravenous, injection. The conjugate according to the invention or the pharmaceutical composition according to the invention may be administered as a single dose or in multiple doses. The mode of administration, dosage and optimum pharmaceutical forms can be determined according to the criteria generally taken into account in the establishment of a treatment adapted to a patient such as, for example, the age or the body weight of the patient, the seriousness of his/her general condition, the tolerance to the treatment and the secondary effects noted. For example, the drug-conjugate according to the invention or the pharmaceutical composition according to the invention may be administered between every 6 hours and every month, preferably every day or every week, more preferably twice a week. The duration of treatment with a drug-conjugate according to the invention or a pharmaceutical composition according to the invention, is preferably

comprised between 4 and 52 weeks, preferably between 12 and 26 weeks. Alternatively, the treatment may last as long as the symptoms of the disease persists.

Cancer

The term "cancer" or "tumor", as used herein, refers to the presence of cells possessing characteristics typical of cancer-causing cells, such as uncontrolled proliferation, and/or immortality, and/or metastatic potential, and/or rapid growth and/or proliferation rate, and/or certain characteristic morphological features. This term refers to any type of malignancy (primary or metastases) in any type of subject. It may refer to solid tumor as well as hematopoietic tumor.

Preferably, the cancer according to the invention is selected from the group consisting of prostate cancer, lung cancer, the breast cancer, gastric cancer, kidney cancer, ovarian cancer, hepatocellular cancer, osteosarcoma, glioma, melanoma, hypopharynx cancer, esophageal cancer, endometrial cancer, cervical cancer, pancreatic cancer, liver cancer, colon or colorectal cancer, rhabdomyosarcoma, mesothelioma, neuroendocrine tumors, malignant tumor of the muscle, adrenal cancer, thyroid cancer, uterine cancer, skin cancer, bladder cancer, oral squamous cancer, Kaposi sarcoma, head and neck cancer, the lymphoma, the leukemia, cancer of unknown primary, and any drug resistant cancer.

More preferably, the cancer according to the invention is selected from the group consisting of ovarian cancer, breast cancer, lung cancer, colorectal cancer.

In one aspect, the drug-conjugate of this invention or pharmaceutical compositions may be employed, for use thereof in the prevention and/or treatment of metastases.

In one aspect, the drug-conjugate of this invention or pharmaceutical compositions may be employed as second or subsequent line therapy in the treatment of cancer.

**EXAMPLES**

This invention will be better understood in light of the following examples, which are provided for illustrative purposes only.

**Example 1 : Preparation of A9-mc-Val-Cit-PAB- conjugates**

Linker [4-[[(2S)-5-(carbamoylamino)-2-[[(2S)-2-[6-(2,5-dioxopyrrol-1-yl)hexanoylamino]-3-methylbutanoyl]amino]pentanoyl]amino]phenyl]methyl (4-nitrophenyl) carbonate (more simply described as maleimido-hexyl-valyl-citrullyl-carbonate-paranitrophenol) was purchased (Tebu-bio, 39 rue de Houdan - BP 15, 78612 Le Perray-en-Yvelines Cedex, France).

It was reacted with each of the 6 drug payloads (21-aminoepothilone, HTI-042, mitomycin A, ombrabulin, DAEDOX, CBACOL) in the following conditions :

- drug 0.25 M (1 eq)
- linker 0.3 M (1.2 eq)
- N,N-Diisopropylethylamine (0-1%)
- pyridine (10%)
- in dimethylsulfoxide (DMSO) as solvent
- room temperature for 6 hours

Each drug-linker was purified by high-performance liquid chromatography (HPLC) and lyophilized.

The peptide A9 has sequence QDVNTAVAW and is known to bind HER2 even with a N-terminal cargo (Honarvar et al. Scientific Reports volume 8, Article number: 2998, 2018). A modified peptide with sequence CGSGQDVNTAVAW-$NH_2$, with a N-terminal cysteine for conjugation and a GSG spacer, and an amide in C-terminal was synthesized using solid-phase peptide synthesis (Smartox Biotech, Grenoble, France).

Each of the 6 linker-drug conjugates was reacted with peptide A9 using the following steps :

- dissolution of the peptide in degassed PBS at 0.1 M (1 eq)
- treatment with tris-carboxyethylphosphine at 2 M (20 eq) 1h at room temperature
- dissolution of 1 eq of drug-linker in DMSO at 0.02 M
- mix peptide solution with drug-linker solution, 1:5 vol/vol, 10-20h at room temperature
- purify by HPLC.

**Example 2 : Preparation of a linker-drug structure L-D whose -LC-LSI- group is from formula (XV) where the linker and drug are bound through a hydroxy group on the drug**

A linker-drug structure L-D whose -$L_C$-$L_{SI}$- group is from formula (XV), and where the linker and drug are bound through a hydroxy group on the drug, can be obtained by using the method described in EP 3 342 785 B1, paragraphs [0283] to [0289], starting from the method described in Japanese Patent Laid-Open No. 2002060351 or the literature (J. Org. Chem., volume 51, page 3196, 1986), and if necessary, by removing the protecting groups or modifying the functional groups.

Once the linker-drug structure L-D is obtained, a process similar to the one described in Example 1 can be used to obtain the full conjugate Bd-L-D.

**Example 3: A linker-drug structure L-D whose -$L_C$-$L_{SI}$- group is from formula (XVI) or formula (XVII), and where the linker and drug are bound through a hydroxy** group **on the drug**

A linker-drug structure L-D whose -$L_C$-$L_{SI}$- group is from formula (XVI) or formula (XVII), and where the linker and drug are bound through a hydroxy group on the drug, can be obtained by using the method described in the literature (Kern et al. J Am Chem Soc, volume 138, no 4, pages 1430-1445, 2016), and if necessary, by adding protecting groups or modifying the functional groups.

Once the linker-drug structure L-D is obtained, a process similar to the one described in Example 1 can be used to obtain the full conjugate Bd-L-D.

## Claims

**1.** A drug conjugate of the following formula (I):

Bd(L-D)$_n$ (I)

wherein,
D is a drug moiety deriving from a cytotoxic drug selected from the group consisting of cytotoxic drugs of the following formula (II) to (VII):

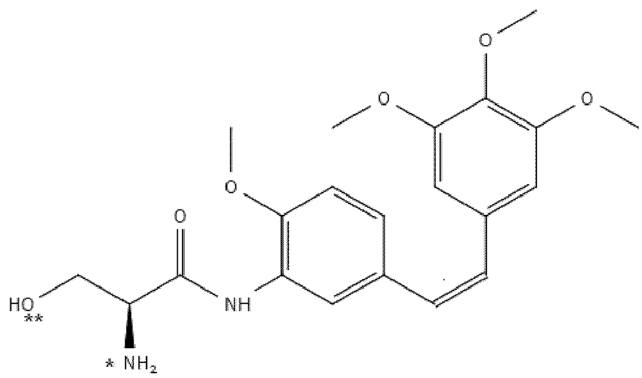

(II);

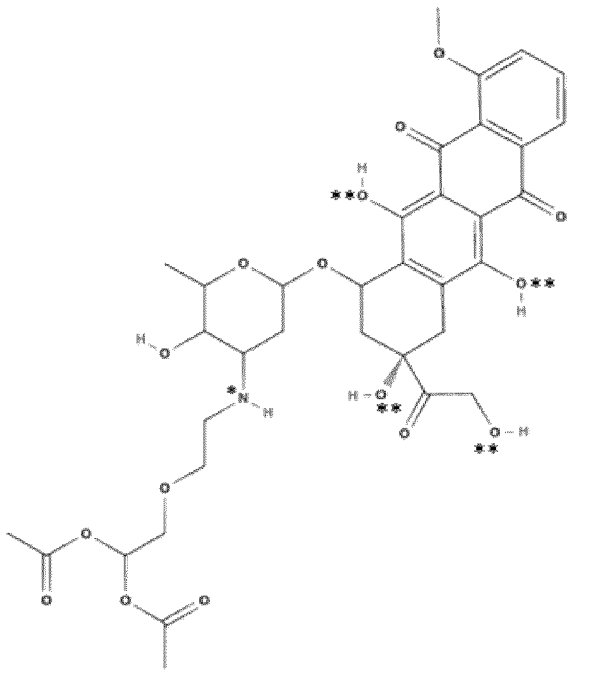

(III);

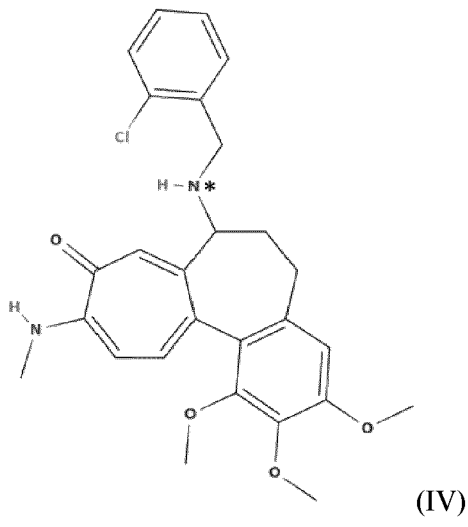

(IV);

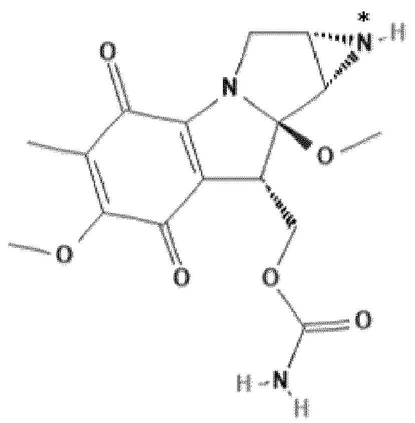

(V);

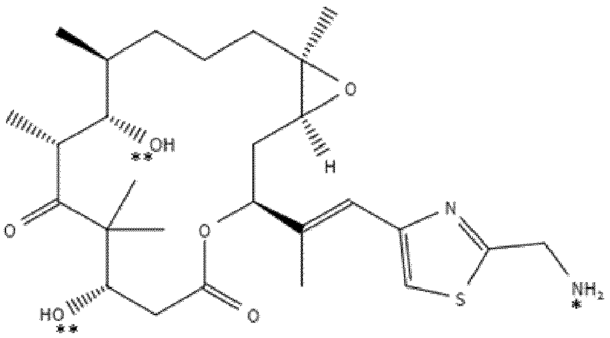

(VI);

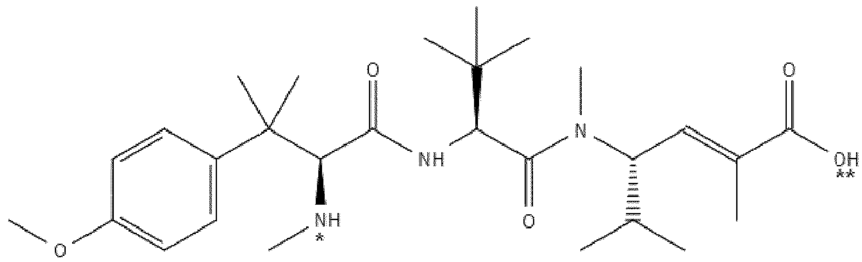

(VII),

wherein the point of attachment with L is the nitrogen atom of the amino group marked with * or the oxygen atom of the hydroxyl group marked with **, and derivatives thereof,
L is a cleavable linker comprising a self-immolative moiety ($L_{SI}$) and an enzyme-cleavable unit ($L_C$); Bd is a binder selected from the group consisting of the molecules that bind to a targeted antigen such as an antigen selected from the group consisting of proteins, glycoproteins, glycolipids, carbohydrates, or a combination thereof; and
n is an integer comprised from 1 to 4;
or a pharmaceutically acceptable salt thereof.

2. The drug conjugate of claim 1, wherein L is of the following formula (VIII):

$$*(-(L_A)_a-(L_S)_s)_r-L_C-L_{SI}-** \quad (VIII),$$

wherein Bd is linked to $-L_A-$ via the bond identified with *, and D is linked to $-L_{SI}-$ via the bond identified with **; and wherein
$-L_A-$ represents an anchor unit, a being 0 or 1;
$-L_S-$ represents a spacer unit, s being 0 to 15;
r is 0 to 10
$-L_C-$ represents an enzyme-cleavable unit; and
$-L_{SI}-$ represents a self-immolative moiety.

3. The drug conjugate of claim 1 or 2, wherein $-L_{SI}-$ is selected form the group consisting of residue of *p*-aminobenzyl alcohol (PAB) unit, residue of 2-aminoimidazol-5-methanol derivatives, hemithioaminal derivatives of para-carboxybenzaldehyde or glyoxilic acid, bis-amine cyclizers, lactam cyclizers, and *ortho*- or *para*-aminobenzylacetals, advantageously $L_{SI}$ is a residue of *p*-aminobenzyl alcohol (PAB) unit.

4. The drug conjugate of claim 1 or 2, wherein $-L_{SI}-$ is a p-aminobenzylcarbonyl unit of the following formula (IX):

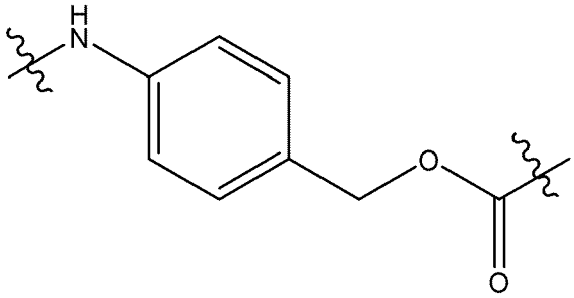
(IX),

wherein $-L_{SI}-$ is linked to $-L_C-$via the aniline group and $-L_{SI}-$ is linked to D via the carbonyl group.

**5.** The drug conjugate of any one of claims 1 to 4, wherein $-L_C-$ is an enzyme cleavable unit, that is cleavable by cathepsin enzyme or plasmin, in particular cathepsin B, cathepsin C or cathepsin D enzyme, preferably cathepsin B enzyme.

**6.** The drug conjugate of any one of claims 1 to 5, wherein $-L_C-$ comprises at least one, preferably from two to four amino acid units, selected from the group consisting of glycine (Gly), valine (Val), citrulline (Cit), phenylalanine (Phe), alanine (Ala) and arginine (Arg), advantageously -LC- is -Ala-Ala-, -Val-Ala-, -Val-Cit-, -Val-Arg-, -Phe-Cit-, -Phe-Arg-; -Ala-Cit, -Ala-Arg-, -Phe-Leu-, -Phe-Lys-, - Gly-Phe-Leu-Gly-, or -Gly-Gly-Phe-Gly-, more advantageously $-L_C-$ is -Val-Cit-.

**7.** The drug conjugate of claim 2, wherein the $-L_C-L_{SI}-$ unit is selected from the group consisting of units of the following formula:

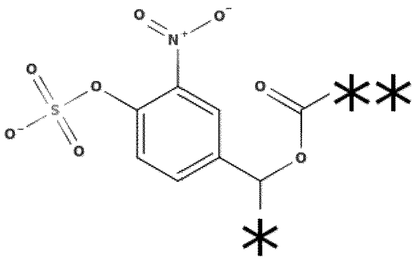
(X)

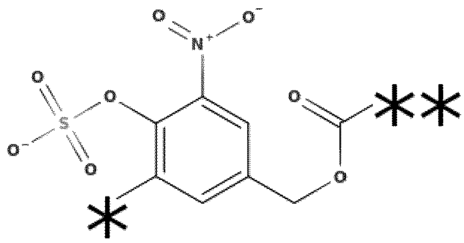
(XI)

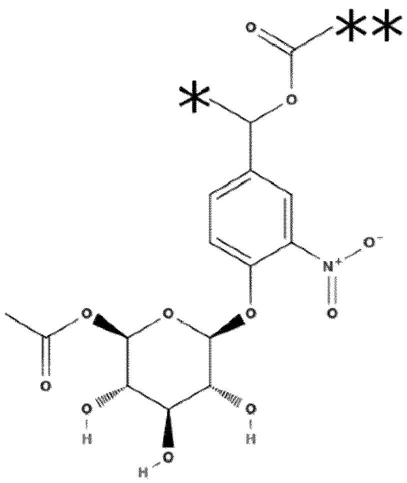
(XII)

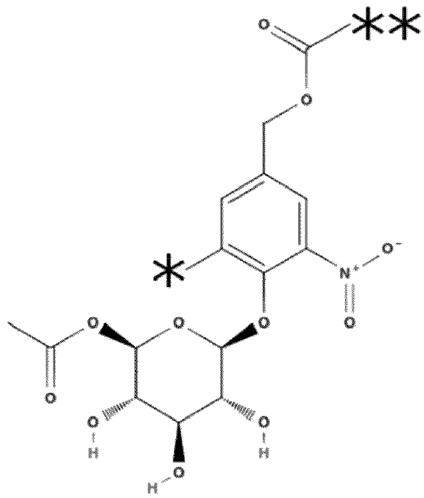
(XIII)

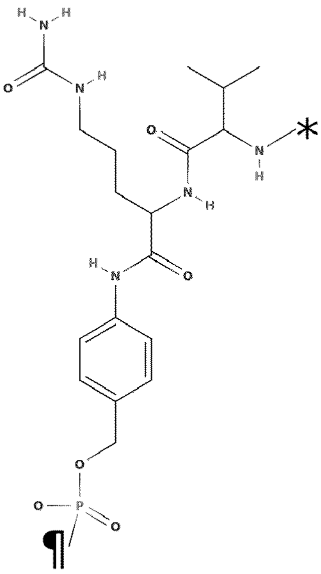

(XIV

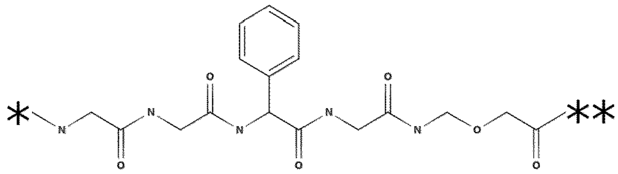

(XV)

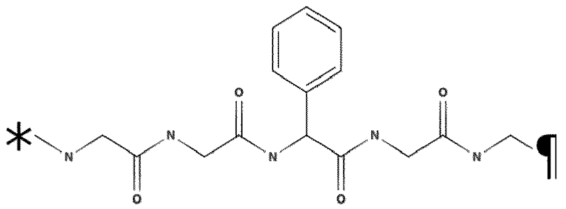

(XVI)

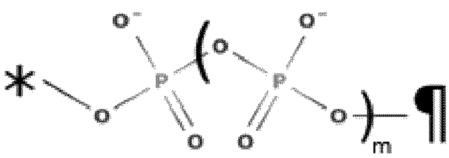

(XVII)

wherein, the nitro ($-NO_2$) group, when present, may be replaced with a hydrogen atom (H), and wherein * refers to the unit $Bd-((L_A)_a-(L_S)_s-)_r$, ** refers to either the payload (-D) or the payload with an additional self-immolative unit ($-L_{SI}'-D$) bound through an amine group, refers to either the payload (-D) or the payload with an additional self-immolative unit ($-L_{SI}'-D$) bound through a hydroxy group, when one is present, and wherein m is 0 to 3.

**8.** The drug conjugate of any one of claims 2 to 7, wherein $-L_S-$ represents a unit selected in the group consisting of sugar moieties, ethylene glycol, aliphatic chain comprising from 0 to 30 carbon atoms, 1-amino-1-glucitolyl-y-glutamate, amino-acids, and homo- and hetero-polymers thereof, either linear or branched, advantageously $-L_S-$ represents a unit selected in the group consisting of 0 to 15 ethylene glycol units and/or 0 to 15 sarcosine units.

**9.** The drug conjugate of any one of claims 2 to 8, wherein $-L_A-$ is or comprises a unit selected in the group consisting of maleimidocaproyl, amide, alkane diamides, alkanedioic acid esters, triazole, disulfide, oxime, hydrazone.

**10.** The drug conjugate of any one of claims 1 to 9, wherein the binder is selected from the group consisting of a antibodies, antiody fragments, peptides, and affibodies.

**11.** The drug conjugate of any one of claims 1 to 10, wherein the binder is selected from the group consisting of a (7-25)amino-acid peptide, preferably a (10-20)amino-acid peptide, preferably a cyclic (10-20)amino-acid peptide, more preferably a cyclic (10-20)amino-acid peptide with a non-proteinogenic amino acid in C or N-terminal, in particular in C-terminal.

**12.** The drug conjugate of any one of claims 1 to 11, wherein the drug is selected from the group consisting of drug of formula (V) or (VI) as defined in claim 1, in particular of formula (VI) as defined in claim 1.

**13.** A drug conjugate as defined in any one of claims 1 to 12, for use as a medicament, in particular for treating cancer.

**14.** A pharmaceutical composition comprising the drug conjugate as defined in any one of claims 1 to 12, and a pharmaceutically acceptable excipient.

**15.** A pharmaceutical composition as defined in claim 14, for use for treating cancer.

Europäisches Patentamt
European Patent Office
Office européen des brevets

# EUROPEAN SEARCH REPORT

Application Number
EP 21 30 5529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 952 394 A (SENTER PETER D [US]) 28 August 1990 (1990-08-28)<br>* figures 1-2 *<br>* column 2, line 41 - column 3, line 42 *<br>* column 3, line 49 *<br>* column 5, line 35 - column 7, line 40 *<br>* column 7, line 41 - line 43 *<br>----- | 1,2,8-15 | INV.<br>A61K47/64<br>A61K47/65<br>A61K47/68<br>A61P35/00 |
| X | US 2007/276018 A1 (VITE GREGORY D [US] ET AL) 29 November 2007 (2007-11-29)<br>* paragraph [0289] - paragraph [0291] *<br>* figure 1 *<br>----- | 1,2,<br>8-10,<br>12-15 | |
| X | WO 2014/096551 A1 (GLYKOS FINLAND OY [FI]) 26 June 2014 (2014-06-26)<br>* page 1, line 20 - line 24 *<br>* page 36, line 21 - page 37, line 31 *<br>* examples 3, 9, 10 *<br>----- | 1-15 | |
| X | US 6 214 345 B1 (FIRESTONE RAYMOND ARMAND [US] ET AL) 10 April 2001 (2001-04-10)<br>* page 3, line 35 - line 54 *<br>* page 4, line 35 - line 45 *<br>* page 6, line 18 - line 32 *<br>* page 9, line 45 *<br>* page 10, line 1 *<br>* page 10, line 17 - line 49 *<br>* schemes 13 and 17 *<br>* page 19, line 3 - page 23, line 37 *<br>----- | 1-15 | TECHNICAL FIELDS SEARCHED (IPC)<br>A61K<br>A61P |

The present search report has been drawn up for all claims

1

| Place of search | Date of completion of the search | Examiner |
|---|---|---|
| The Hague | 6 October 2021 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 30 5529

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2021

| Patent document cited in search report | Publication date | Patent family member(s) | Publication date |
|---|---|---|---|
| US 4952394 A | 28-08-1990 | AT 81986 T | 15-11-1992 |
| | | AU 2582488 A | 25-05-1989 |
| | | CA 1303524 C | 16-06-1992 |
| | | DE 3875700 T2 | 18-03-1993 |
| | | DK 651088 A | 24-05-1989 |
| | | EP 0317957 A2 | 31-05-1989 |
| | | FI 885361 A | 24-05-1989 |
| | | GR 3006211 T3 | 21-06-1993 |
| | | JP H02111731 A | 24-04-1990 |
| | | KR 890007724 A | 05-07-1989 |
| | | NO 169958 B | 18-05-1992 |
| | | NZ 226955 A | 26-11-1991 |
| | | PT 89062 A | 30-11-1989 |
| | | US 4952394 A | 28-08-1990 |
| | | ZA 886811 B | 26-07-1989 |
| US 2007276018 A1 | 29-11-2007 | AR 061181 A1 | 13-08-2008 |
| | | AT 524477 T | 15-09-2011 |
| | | AU 2007267535 A1 | 06-12-2007 |
| | | BR PI0712167 A2 | 28-08-2012 |
| | | CA 2655668 A1 | 06-12-2007 |
| | | CN 101495482 A | 29-07-2009 |
| | | EA 200802389 A1 | 30-06-2009 |
| | | EP 2041140 A2 | 01-04-2009 |
| | | ES 2371111 T3 | 27-12-2011 |
| | | HK 1124335 A1 | 10-07-2009 |
| | | IL 195236 A | 31-12-2012 |
| | | JP 5249929 B2 | 31-07-2013 |
| | | JP 2009538349 A | 05-11-2009 |
| | | KR 20090025266 A | 10-03-2009 |
| | | NZ 572763 A | 27-08-2010 |
| | | PE 20080316 A1 | 10-04-2008 |
| | | TW 200815452 A | 01-04-2008 |
| | | US RE42930 E | 15-11-2011 |
| | | US 2007276018 A1 | 29-11-2007 |
| | | WO 2007140297 A2 | 06-12-2007 |
| | | ZA 200810022 B | 25-05-2011 |
| WO 2014096551 A1 | 26-06-2014 | EP 2934596 A1 | 28-10-2015 |
| | | US 2015314007 A1 | 05-11-2015 |
| | | WO 2014096551 A1 | 26-06-2014 |
| US 6214345 B1 | 10-04-2001 | AT 212236 T | 15-02-2002 |
| | | AU 687795 B2 | 05-03-1998 |
| | | CA 2123363 A1 | 15-11-1994 |
| | | CN 1100426 A | 22-03-1995 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 30 5529

This annex lists the patent family members relating to the patent documents cited in the above-mentioned European search report. The members are as contained in the European Patent Office EDP file on
The European Patent Office is in no way liable for these particulars which are merely given for the purpose of information.

06-10-2021

| Patent document cited in search report | Publication date | Patent family member(s) | Publication date |
|---|---|---|---|
| | | DE 69429689 T2 | 17-10-2002 |
| | | DK 0624377 T3 | 06-05-2002 |
| | | EP 0624377 A2 | 17-11-1994 |
| | | ES 2170755 T3 | 16-08-2002 |
| | | FI 942237 A | 15-11-1994 |
| | | HU 224351 B1 | 29-08-2005 |
| | | JP 3645283 B2 | 11-05-2005 |
| | | JP H0770175 A | 14-03-1995 |
| | | NO 315162 B1 | 21-07-2003 |
| | | NZ 260512 A | 25-09-1996 |
| | | PT 624377 E | 31-07-2002 |
| | | RU 94016384 A | 10-11-1996 |
| | | US 6214345 B1 | 10-04-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015063331 A
- US 20030187039 A1
- US 8129407 B2
- ER 807842
- ER 806005
- ER 806073
- WO 2003084919 A2
- EP 3342785 B1
- JP 2002060351 A


### Non-patent literature cited in the description


- **TAE JIN KIM et al.** *Cancer Research,* 01 October 2007, vol. 67 (19
- **DAVID FARQUHAR et al.** *J. Med. Chem.,* 1998, vol. 41, 965-972
- **JULIA KRZYWIK.** *Molecules,* 2020, vol. 25, 3540
- **KENNETH R. KUNZ et al.** *J. Med. Chem.,* 1991, vol. 34, 2281-2286
- **ROBERT T. DORR.** *Cancer Chemother Pharmacol,* 1992, vol. 31, 1-5
- **C. SESSA.** *Annals of Oncology,* 2007, vol. 18, 1548-1553
- **ZASK et al.** *J Med Chem,* 2004, vol. 47, 4774-4786
- *CHEMICAL ABSTRACTS,* 253426-24-3
- *CHEMICAL ABSTRACTS,* 4055-39-4
- *CHEMICAL ABSTRACTS,* 280578-49-6
- *Polym. Chem.,* 2011, vol. 2, 773-790
- **YVES MEYER et al.** *Org. Biomol. Chem.,* 2010, vol. 8, 1777-1780
- **STEPHANOPOULOS ; FRANCIS.** *Nat. Chem. Biol.,* 15 November 2011, vol. 7 (12), 876-84
- **KALIA ; RAINES.** *Curr Org Chem.,* January 2010, vol. 14 (2), 138-147
- **OLAFSEN et al.** *Protein Eng. Design & Sel.,* 2004, vol. 17 (4), 315-323
- **HUSE et al.** *Science,* 1989, vol. 246, 1274-1281
- **NIU et al.** *Bioorg Med Chem Lett,* 2010, vol. 20, 1535-1538
- **VYAS et al.** *J. Org. Chem.,* 1986, vol. 51 (22), 4307-4309
- **FARQUHAR et al.** *J. Med. Chem.,* 1998, vol. 41, 965-972
- **KRZYWIK et al.** *Molecules,* 2020, vol. 25, 3540
- *J. Org. Chem.,* 1986, vol. 51, 3196
- **KERN et al.** *J Am Chem Soc,* 2016, vol. 138 (4), 1430-1445

## SEQUENCE LISTING

```
<110>  Centaurus Polytherapeutics

<120>  Novel payloads for drug-conjugates and their use for treating
       cancer

<130>  B3525EP00

<160>  2

<170>  PatentIn version 3.5

<210>  1
<211>  9
<212>  PRT
<213>  Artificial Sequence

<220>
<223>  A9 peptide

<400>  1

Gln Asp Val Asn Thr Ala Val Ala Trp
1               5


<210>  2
<211>  13
<212>  PRT
<213>  Artificial Sequence

<220>
<223>  peptide


<220>
<221>  MISC_FEATURE
<222>  (13)..(13)
<223>  Amide group (NH2)

<400>  2

Cys Gly Ser Gly Gln Asp Val Asn Thr Ala Val Ala Trp
1               5                   10
```

SEQUENCE LISTING

<110> Centaurus Polytherapeutics (SAS)

<120> Novel payloads for drug-conjugates and their use for treating cancer

<130> PR2584EP00

<160> 4

<170> PatentIn version 3.5

<210> 1
<211> 9
<212> PRT
<213> Artificial Sequence

<220>
<223> A9 peptide

<400> 1

```
Gln Asp Val Asn Thr Ala Val Ala Trp
1               5
```

<210> 2
<211> 13
<212> PRT
<213> Artificial Sequence

<220>
<223> peptide

<220>
<221> MISC_FEATURE
<222> (13)..(13)
<223> Amide group (NH2)

<400> 2

```
Cys Gly Ser Gly Gln Asp Val Asn Thr Ala Val Ala Trp
1               5                   10
```

<210> 3
<211> 4
<212> PRT
<213> Artificial Sequence

<220>
<223> -Lc-

<400> 3

```
Gly Phe Leu Gly
1
```

<210> 4
<211> 4

<212> PRT
<213> Artificial Sequence

<220>
<223> -Lc-

<400> 4

Gly Gly Phe Gly
1